# FASCICULE DE BREVET EUROPEEN

(11) **EP 0 516 803 B1**
(45) Date de publication et mention de la délivrance du brevet: **20.12.1995**
(21) Numéro de dépôt: 92902195.4
(22) Date de dépôt: 17.12.1991
(51) Int. Cl.: C12P 19/12, C12P 19/44, C12R 1/72, C12R 1/88

(54) **PROCEDE DE PRODUCTION DE SOPHOROSIDES PAR FERMENTATION AVEC ALIMENTATION CONTINUE EN ESTERS D'ACIDES GRAS OU EN HUILES**
VERFAHREN ZUR HERSTELLUNG VON SOPHOROSIDEN DURCH FERMENTATION MIT KONTINUIERLICHE ZUFÜHRUNG VON FETTSÄUREESTERN UND/ODER ÖLEN
METHOD OF PRODUCTION OF SOPHOROSIDES BY FERMENTATION WITH FED BATCH SUPPLY OF FATTY ACID ESTERS OR OILS

(30) Priorité: 20.12.1990 FR 9016211
(43) Date de publication de la demande: 09.12.1992
(73) Titulaire: INSTITUT FRANCAIS DU PETROLE, 92506 Rueil-Malmaison Cédex (FR)
(72) Inventeur: MARCHAL, Rémy, F-78400 Chatou (FR); LEMAL, Jeannine, F-92500 Rueil-Malmaison (FR); SULZER, Caroline, F-92500 Rueil-Malmaison (FR)
(74) Mandataire: Andreeff, François
(86) Numéro de dépôt international: FR9101027
(87) Numéro de publication internationale: WO9211381

(56) Documents cités:
- JOURNAL OF THE AMERICAN OIL CHEMISTS SOCIETY vol. 65, no. 9, Septembre 1988, CHAMPAIGN, ILLINOIS, USA pages 1460 - 1466; HANS-JOACHIM ASMER ET AL.: 'Microbial production, structure elucidation and bioconversion of sophorose'

## Description

L'invention concerne un procédé de production avec alimentation continue du substrat (fed-batch) d'une composition de sophorosides par fermentation. Les sophorosides sont par exemple utilisés en cosmétologie, dans le traitement des cheveux contre les pellicules et comme agent bactériostatique dans les déodorants, notamment sous la forme lactone EP-B-209783.

Il a été mentionné dans les brevets US 32O5150 et US 3312684 qu'une quantité de sophorosides était produite par un procédé de fermentation mettant en jeu une culture de *Torulopsis bombicola*, souche actuellement classée *Candida bombicola*.

Les sophorosides sont considérés comme étant un mélange de composés représentés par les formules (1) et (2). 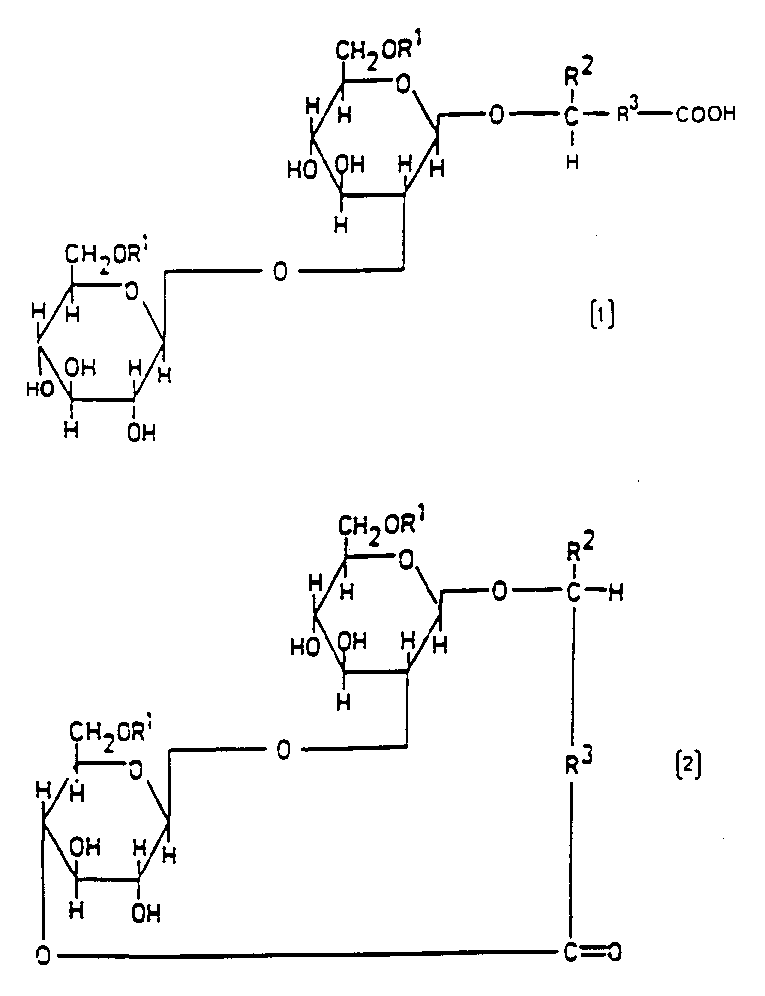
dans laquelle R¹ représente l'hydrogène ou un groupe acétyle et R² l'hydrogène ou un reste alkyle comportant 1 à 9 atomes de carbone, lorsque R³ est un reste hydrocarboné saturé à 7 à 16 atomes de carbone, ou bien R² représente l'hydrogène ou un groupe méthyle, lorsque R³ est un reste hydrocarboné insaturé à 13 à 17 atomes de carbone.

Ces composés sont utilisables comme agents de nettoyage et comme émulsifiants et ils présentent d'excellentes propriétés hygroscopiques et hydrophiles dues au groupement sophorose et des propriétés hydrophobes provenant de l'acide gras.

Toutefois, les sophorosides sont un ensemble de nombreux homologues et le rapport de formation de ces homologues varie en fonction de leur substrat, une source hydrocarbonée par exemple, et des conditions de fermentation (FR 2399438).

Par conséquent, les propriétés et les fonctions de ces composés varient avec les rapports des homologues compte tenu du fait qu'on utilise généralement un ensemble de ces homologues et il s'avère jusqu'à présent difficile de pouvoir produire un produit avec un rapport donné par un procédé de fermentation.

L'art antérieur peut être illustré par le doucment. "Journal of the american oil chemists society vol 65, No. 9 Septembre 1988, Champaign, Illinois, USA pages 1460-1466 : H.J. ASMER et al. - "Microbial production, structure elucidation and bioconversion of sophorose lipids."

La préparation des sophorosides est généralement effectuée en présence d'un substrat tel que décrit dans le brevet US 3205150. Par exemple, ce substrat peut être des hydrocarbures, des acides gras saturés ou insaturés, des esters d'acides incluant des glycérides, des huiles végétales telles que l'huile de soja.

L'alimentation est habituellement réalisée en discontinu à des intervalles de temps d'environ 12 à 24 heures, avec une quantité d'environ 2 % en poids par rapport au volume réactionnel initial, pour chaque addition.

Il est précisé que la présence de quantité de substrat supérieure ( 3 à 4 % environ) contribue à une diminution du rendement des sophorosides produits.

Par ailleurs, vingt quatre à quarante huit heures après la dernière addition de substrat, il n'est observé aucune conversion supplémentaire.

Ce procédé de production en batch est aussi décrit dans le brevet US 4297340 où un ajout d'une quantité de substrat (150 g) est apporté toutes les 24 heures pendant six jours, la durée totale de production de la culture étant de sept jours.

Ces procédés d'alimentation en discontinu permettent d'atteindre des productions finales en sophorosides bruts ne dépassant pas 23 % et n'ont, par conséquent, que des productivités limitées. La cause de cette limitation peut être recherchée dans les capacités lipolytiques du microorganisme qui transforme les huiles ou les esters résiduels du milieu de fermentation en acides gras. Ces acides gras ne sont pas inhibiteurs pour la croissance du microorganisme mais ils sont susceptibles d'affecter de façon sensible la vitesse de production des sophorosides lorsque ceux-ci sont déjà présents dans le milieu.

L'objet de l'invention est donc de remédier aux inconvénients de l'art antérieur.

On a découvert un procédé de préparation des spohorosides permettant notamment d'améliorer la productivité de la souche cultivée, le rendement en sophorosides produit et de ce fait la production en sophorosides recherchés.

De manière plus détaillée, l'invention concerne un procédé de production en fed batch d'une composition de sophorosides, dans lequel on met en culture au moins une souche *Candida bombicola* ou *Candida apicola* dans un milieu de culture comprenant une source d'azote dans des conditions appropriées telles que l'on produit ladite souche et l'on soumet ladite souche cultivée dans une zone de réaction à une alimentation de préférence excédentaire en sucre et à une alimentation en continu en au moins un substrat approprié dans des conditions d'aération, de température, de pH, adéquates, caractérisé en ce qu'on réalise au moins une fois la séquence suivante :
*a*. On effectue l'alimentation de la souche en ledit substrat à un débit d'alimentation dans la zone réactionnelle comprise entre 0,01 et 4 grammes par heure et par litre de volume réactionnel initial et pendant une durée d'alimentation tels que la concentration résiduelle en ledit substrat dans la zone réactionnelle soit maintenue à une valeur au plus égale à 18 grammes par litre de volume réactionnel initial pendant ladite durée d'alimentation ; et
*b*. On récupère la composition de sophorosides produits.

De manière préférée, l'alimentation en continu en substrat peut s'effectuer selon un profil décroissant au cours du temps.

Selon une caractéristique du procédé, l'étape de récupération de la composition de sophorosides comprend l'arrêt de l'alimentation en substrat ou de l'aération de la zone réactionnelle, la récupération après décantation de la phase inférieure sophoroside contenant de l'eau et au moins un lavage à l'eau des sophorosides à une température comprise en général entre 10 et 90°C pendant 10 à 60 mn par exemple.

La souche utilisée est avantageusement *Candida bombicola* CBS 6009. La productivité observée est alors excellente et le rendement en sophorosides produits est très supérieur à celui de l'art antérieur.

La mise en oeuvre du procédé peut être réalisée de la façon suivante :
- suivant un premier mode, on alimente en substrat en régulant le débit d'alimentation dans la zone réactionnelle dans les conditions opératoires précitées, on arrête l'alimentation en substrat lorsque la quantité totale de substrat injecté atteint environ au plus 280 g.l⁻¹ de volume réactionnel initial et on récupère la composition de sophorosides comme il a été indiqué ci-dessus ;
- suivant un deuxième mode plus avantageux, on alimente en substrat en régulant le débit d'alimentation dans la zone réactionnelle dans des conditions telles que la concentration résiduelle en substrat soit voisine d'une valeur préalablement fixée comprise en général entre 0,1 et 18 g.l⁻¹ et de préférence entre 0,5 et 3 g.l⁻¹. Dès que la concentration en O₂ dans la zone réactionnelle dissout devient de manière avantageuse proche de zéro, on stoppe l'alimentation en substrat et l'agitation du fermenteur, on fait décanter la solution, on récupère la phase sophoroside que l'on lave au moins une fois à une température comprise entre 10 et 90°C et on alimente à nouveau en substrat la zone réactionnelle débarrassée de cette phase sophoroside. On peut ainsi recommencer cette séquence d'alimentation décantation et récupération jusqu'à ce que les capacités de biosynthèse des microorganismes soient épuisées.

Selon une autre caractéristique du procédé et de l'alimentation en continu, la zone de réaction peut contenir initialement éventuellement un sucre en excès et une concentration en substrat de 0,5 à 40 g.l⁻¹ de volume réactionnel initial, avantageusement de 1 à 25 g.l⁻¹ et on procède à l'alimentation en continu de ladite souche en substrat après une période, par exemple, d'au plus 48 heures, c'est-à-dire lorsque la concentration initiale en substrat est généralement comprise entre O,1 et 15 g.l⁻¹ par litre de milieu réactionnel initial et de préférence entre 0,1 et 5 g.l⁻¹.

Selon une autre caractéristique avantageuse du procédé permettant d'obtenir de bons résultats, le débit d'alimentation en continu en substrat dans la zone réactionnelle peut être compris entre 1,0 et 3,0 g.h⁻¹.l⁻¹ de volume réactionnel initial et de façon préférée entre 2,0 et 2,5 g.h⁻¹.l⁻¹.

Ce substrat comprend habituellement au moins une huile animale, au moins une huile végétale et/ou au moins un ester de ladite huile, lesdites huiles et lesdits esters incorporant une chaîne linéaire aliphatique (saturée ou insaturée) de 10 à 24 atomes de carbone.

Parmi les huiles et esters préférés, on peut citer : l'huile et les esters méthyliques ou éthyliques de l'huile de colza, de tournesol, de palme ou de soja.

On a obtenu d'excellents résultats avec lesdits esters.

Le milieu de culture peut comprendre une source d'azote minéral (sous forme d'ions ammonium) et/ou azote organique, par exemple sous forme d'aminoacides tels que notamment l'extrait de levure, la peptone de soja, des hydrolysats de caséine, de la liqueur de macération de maïs, des hydrolysats de gluten de blé, des extraits de viande. L'addition d'éléments minéraux tels que par exemple le potassium, le sodium, le magnésium et d'oligoéléments comme le fer, le manganèse, le molybdène sous forme de leurs sels (sulfates, phosphates, chlorures) peuvent permettre également d'améliorer encore la croissance.

Le milieu de culture peut comprendre au moins un sucre tel que le glucose ou le saccharose.

Les conditions de culture sont habituellement les suivantes : température : 18-35°C ; pH : 3,0 à 8,0. Un bon niveau d'activité a été obtenu à une température comprise entre 20 et 30°C et dans un intervalle de pH de 3,0 à 5,0 et on observe d'excellents niveaux d'activité à une température comprise entre 22 et 28°C et à un pH de 3,5 à 4.0. La fermentation s'effectue en conditions initiales d'asepsie et en aérobiose.

Selon une autre caractéristique du procédé, on peut introduire la souche contenue dans le milieu de culture dans la zone réactionnelle afin de la soumettre à l'alimentation en sucre et en substrat, mais on peut aussi selon une autre caractéristique du procédé soutirer la souche du milieu de culture par des techniques connues de l'Homme de métier et l'introduire dans la zone réactionnelle où on la soumet à l'alimentation en sucre et en substrat.

Le procédé de production des sophorosides est en règle générale mis en oeuvre dans les conditions suivantes : température : 18 à 35°C ; pH : 2,5 à 8,0, avantageusement 3,0 à 4,0 ; débit d'aération : 0,2 à 2 VVM sous une pression de 1 à 5 bar et de préférence 1 à 2 bar (1 bar = 0,1 MPa).

Pendant toute la durée de la production, le pH est contrôlé et régulé à une valeur de consigne comprise dans la gamme décrite plus haut, par l'apport d'une solution de soude ou de potasse par exemple.

La quantité de cellules mises en jeu par rapport au volume réactionnel initial est habituellement de 1 g à 100 g de poids sec par litre et de préférence de 10 à 30 g de poids sec par litre.

L'invention sera mieux comprise au vu des exemples illustratifs suivants :

### EXEMPLE 1

Cet exemple décrit une fermentation effectuée selon l'invention. La souche *Candida bombicola* CBS 6009 est utilisée pour ensemencer un milieu qui, indépendamment du glucose, est dépourvu de substrat. Celui-ci est ajouté en continu après l'ensemencement. Il s'agit de l'ester éthylique de colza.

Le milieu de culture utilisé est le suivant :

| | |
|---|---|
| glucose | 100 g.l⁻¹ |
| (NH₄)₂SO₄ | 4 g.l⁻¹ |
| KH₂PO₄ | 1 g.l⁻¹ |
| MgSO₄, 7H₂0 | 0,5 g.l⁻¹ |
| Liqueur séchée de macération de maïs | 5,0 g.l⁻¹ |

Glucose et MgSO₄, 7H₂0 sont stérilisés dans un fermenteur de laboratoire de 4 litres de capacité en solution dans 1620 ml d'eau tandis que KH₂PO₄, (NH₄)₂SO₄ et liqueur de maïs sont stérilisés séparément dans une fiole d'Erlenmeyer en solution dans 180 ml d'eau. La stérilisation des deux solutions est réalisée à l'autoclave à la température de 120°C pendant 30 minutes et le milieu de culture est reconstitué après refroidissement des deux solutions. Il est ensemencé par 200 ml d'une préculture préparée dans une fiole d'Erlenmeyer avec 200 ml du même milieu que le milieu de fermentation mais additionné de 1 ml d'ester éthylique de colza. Cette fiole est ensemencée par 1 g environ d'un congelat de la souche de *Candida* *bombicola*. Elle est incubée sous agitation à la température de 25°C. Elle fournit après 24 heures la préculture du fermenteur.

La fermentation de production est effectuée dans le fermenteur de quatre litres de capacité sur un volume réactionnel initial de deux litres.

L'agitation du milieu est obtenue par une turbine RAYNERI dont la vitesse de rotation est de 1000 rpm. L'aération est fixée à 0,5 v.v.m d'air sous pression atmosphérique. Après auto-acidification de la culture, le pH du milieu est maintenu à la valeur constante de 3,5 grâce à une solution de soude 4N dont l'addition dans le réacteur est contrôlée par une électrovanne asservie à un pH-mètre. La teneur en oxygène dissous dans le milieu de culture est mesurée en continu grâce à une électrode polarographique reliée à un enregistreur.

Pour éviter toute limitation du milieu en glucose, on procède à cinq additions de ce sucre sous forme solide. Les trois premières aux temps t = 24 h, t = 48 h, t = 72 h correspondant chacune à 45 g de glucose par litre de milieu initial tandis que les deux dernières à t = 96 h et t = 120 h correspondent chacune à 32,5 g de glucose par litre. L'alimentation en ester éthylique de colza est réalisée grâce à une pompe péristaltique dont la vitesse d'alimentation est fixée à raison de 2 g.l⁻¹.h⁻¹ jusqu'au temps t = 96 h puis à raison de 1 g.l⁻¹.h-¹ jusqu'au temps t = 144 h. On suit par des prélèvements d'échantillons la production des sophorosides que l'on mesure par la méthode de Göbbert et al. (1984, Biotechnol. lett. 6, 225-230). Cette production qui commence après la croissance du microorganisme augmente avec l'addition d'ester éthylique de colza. La concentration résiduelle de ce dernier, mesurée par chromatographie en phase gazeuse après extraction à chaud à l'heptane est égale à 0,7 g.l⁻¹.

Après 144 h de fermentation, on arrête l'agitation, l'aération et l'alimentation en ester éthylique pour laisser décanter les sophorosides bruts pendant une heure. On les récupère et on les met en agitation avec 1,5 l d'eau distillée à la température de 45°C. On les laisse à nouveau décanter pendant quatre heures et on effectue un second lavage dans les mêmes conditions. A l'issue du deuxième lavage, on récupère 565 g de sophorosides bruts par litre de milieu initial. La productivité de la fermentation calculée par rapport aux sophorosides bruts et sur la durée de l'essai, est donc égal à 3,92 g de sophorosides bruts par litre de milieu initial et par heure. La teneur en eau des sophorosides bruts mesurée par la méthode de Karl-Fischer est de 45 %. Compte tenu des quantités de sucre et d'ester qui ont été fournies et qui sont égales respectivement à 300 et 240 g.l⁻¹, le rendement de production en sophorosides anhydres par rapport à la somme de glucose plus ester s'élève à 59,5 %. La production, la productivité et les rendements ont été également mesurés après 96 heures de culture (immédiatement avant la quatrième addition de glucose) pour comparaison avec le protocole d'alimentation de l'ester en discontinu (exemple 2). Après 96 heures, la production de sophorosides bruts correspond à 295 g.l⁻¹ et donc à une productivité de 3,07 g.l⁻¹. Le rendement de conversion de "glucose plus ester éthylique" en sophorosides anhydres est alors égal à 38 %.

### EXEMPLE 2 (comparatif)

On recommence l'exemple 1 en remplaçant l'alimentation continue d'ester éthylique de colza par une addition toutes les 12 heures, de 20 g.l⁻¹ d'ester éthylique de colza. La première addition a lieu immédiatement après l'ensemencement du fermenteur. Comme dans l'exemple 1, on suit la cinétique de production des sophorosides bruts dans le milieu de fermentation. Celle-ci débute à la fin de la croissance, mais s'arrête prématurément après 96 heures, en même temps que la 9ème addition d'ester éthylique de colza. Si l'on poursuit les additions d'ester éthylique, on ne peut, de surcroît, plus procéder à la récupération par décantation des sophorosides qui restent intimement mélangés à l'ester éthylique résiduel. Après 96 heures on ne récupère que 210 g.l⁻¹ de sophorosides bruts. La productivité calculée par rapport aux sophorosides bruts et sur la durée de 96 heures est égale à 2,18 g.l⁻¹.h⁻¹. Le rendement de conversion du "glucose (235 g.l⁻¹) plus ester éthylique de colza (180 g.l⁻¹)" en sophorosides anhydres (taux d'humidité : 45 %) est égal à 27,8 %. Ces performances sont inférieures à celles obtenues après la même période de temps dans l'exemple 1.

### EXEMPLE 3

On répète l'exemple 1 jusqu'au temps t = 95 h, heure à laquelle l'épuisement en oxygène dissous du milieu est sensiblement total. Durant cette première période, la concentration d'ester éthylique de colza résiduelle dans le milieu de fermentation est égal à 0,5 g.l⁻¹. On arrête alors l'agitation, l'aération et l'alimentation en ester pendant 15 minutes et on soutire les sophorosides qui ont décanté. On remet en marche l'aération, l'agitation et l'alimentation en ester du fermenteur et l'on poursuit l'essai comme suit : on ajoute 45 g.l⁻¹ de glucose à t = 96 h et t = 120 h puis 32,5 g.l⁻¹ à t = 144 h. A la 96ème heure on réduit la vitesse d'alimentation en ester éthylique de 2 g.l⁻ ¹.h⁻¹ à 1,6 g.l⁻¹.h⁻¹ et l'on poursuit l'alimentation avec cette vitesse jusqu'à t = 144 h. A ce moment, on réduit une nouvelle fois la vitesse d'alimentation en ester à 0,92 g.l⁻¹.h⁻¹ jusqu'à t = 168 h. On procède alors à une seconde récupération de sophoroside par décantation constatant que les capacités de biosynthèse du microorganisme ne sont pas épuisées. Les sophorosides bruts récupérés à t = 95 h et t = 168 h sont rassemblés et lavés deux fois comme dans l'exemple 1. On récupère ainsi 705 g.l⁻¹ de sophorosides bruts après 168 h de culture. La productivité de la fermentation calculée sur la durée de l'essai est de 4,19 g.l⁻¹.h⁻¹ pour une quantité de sucre fournie de 357,5 g.l⁻¹ et une quantité d'ester éthylique de 290 g.l⁻¹. Le rendement en sophorosides anhydres est de 59,8 % par rapport aux deux substrats.

### EXEMPLE 4

On répète l'exemple 1 en ajoutant 24 g.l⁻¹ d'ester éthylique de colza au milieu de culture et on commence l'addition en continu de l'ester éthylique douze heures après l'ensemencement. La vitesse d'injection est fixée à 2 g.l⁻ ¹.h⁻¹ jusqu'à t = 96 h puis 1 g.l⁻¹.h⁻¹ jusqu'à 144 h. Après 144 heures, on récupère 550 g de sophorosides bruts par litre de milieu initial. La productivité par rapport aux sophorosides bruts est de 3,82 g.l⁻¹.h⁻¹ et le rendement en sophorosides anhydres est de 56 %.

### EXEMPLE 5

On répète l'exemple 1 en remplaçant l'ester éthylique de colza par l'ester méthylique de colza. A la fin de l'essai, on récupère 525 g de sophorosides bruts, ce qui correspond à une productivité de 3,64 g.l⁻¹.h⁻¹ de sophorosides bruts et un rendement de 53,4 % de sophorosides anhydres par rapport à la somme "glucose plus ester".

### EXEMPLE 6

On répète l'exemple 1 en remplaçant l'ester éthylique de colza par l'ester méthylique de tournesol. A la fin de l'essai on récupère 505 g.l⁻¹ de sophorosides bruts, ce qui correspond à une productivité de 3,50 g.l⁻¹.h⁻¹ et un rendement de 51,4 % de sophorosides anhydres par rapport à la somme "glucose plus ester méthylique".

### EXEMPLE 7

On répète l'exemple 1 en remplaçant l'ester méthylique de colza par l'huile de colza. La vitesse d'injection d'huile est fixée à 1,2 g.l⁻¹.h⁻¹ jusqu'à t = 96 h puis à 0,60 g.l⁻¹.h⁻¹. Les trois premières additions de glucose aux temps t = 24 h, t = 48 h et t = 72 h correspondent chacune à 30 g.l⁻¹ tandis que les deux dernières à t = 96 h et t = 120 h correspondent chacune à 20 g.l⁻¹ du milieu initial. Après 144 heures par exemple de culture on récupère 380 g de sophorosides bruts ce qui correspond à une productivité de 2,64 g.l⁻¹.h⁻¹ de sophorosides bruts et un rendement de 53 % en sophorosides anhydres (taux d'humidité : 45 %).

## Revendications

1. Procédé de production en fed batch d'une composition de sophorosides, dans lequel on met en culture au moins une souche *Candida bombicola* ou *Candida apicola* dans un milieu de culture comprenant une source d'azote dans des conditions appropriées telles que l'on produit ladite souche et l'on soumet ladite souche cultivée dans une zone de réaction à une alimentation en sucre et a une alimentation en au moins un substrat approprié dans des conditions d'aération, de température, de pH, adéquates, caractérisé en ce qu'on réalise au moins une fois la séquence suivante :
*a*. On effectue en continu l'alimentation de la souche en ledit substrat à un débit d'alimentation dans la zone réactionnelle comprise entre 0,01 et 4 grammes par heure et par litre de volume réactionnel initial et pendant une durée d'alimentation tels que la concentration résiduelle en ledit substrat dans la zone réactionnelle soit maintenue à une valeur au plus égale à 18 grammes par litre de volume réactionnel initial pendant ladite durée d'alimentation ; et
*b*. On récupère la composition de sophorosides produits.

2. Procédé selon la revendication 1, dans lequel l'étape de récupération de la composition de sophorosides comprend l'arrêt de l'alimentation en substrat ou de l'aération de la zone réactionnelle, la récupération après décantation de la phase inférieure sophoroside contenant de l'eau et au moins un lavage à l'eau des sophorosides à une température comprise entre 10 et 90°C pendant 10 à 60 mn.

3. Procédé selon l'une des revendications 1 à 2, dans lequel on stoppe l'alimentation en substrat lorsque la concentration en oxygène dans la zone réactionnelle est sensiblement égale à zéro.

4. Procédé selon l'une des revendications 1 à 2, dans lequel on arrête l'alimentation en substrat lorsque la quantité totale de substrat injecté atteint au plus 280 g.l⁻¹ de volume réactionnel initial.

5. Procédé selon l'une des revendications 1 à 4, dans lequel on met en oeuvre la production de sophorosides à une température de 18 à 35°C, à un pH de 2,5 à 8, en présence d'un excès de sucre et dans lequel on aère la zone de réaction à un débit de 0,2 à 2 v.v.m sous une pression de 1 à 5 bar.

6. Procédé selon l'une des revendications 1 à 5, dans lequel le substrat comprend au moins une huile animale, au moins une huile végétale, et/ou au moins un ester de ladite huile, lesdites huiles et ledit ester incorporant une chaine linéaire aliphatique de 10 à 24 atomes de carbone.

7. Procédé selon l'une des revendications 1 à 6, dans lequel la souche est *Candida bombicola* CBS 6009.

8. Procédé selon l'une des revendications 1 à 7, dans lequel le milieu de culture comprend du sucre.

9. Procédé selon l'une des revendications 1 à 8, dans lequel le débit de substrat dans la zone de réaction est compris entre 1,0 et 3,0 g.l⁻ ¹.h⁻¹ de volume réactionnel initial.

10. Procédé selon l'une des revendications 1 à 9, dans lequel la souche provient d'une culture réalisée ex-situ.

11. Procédé selon l'une des revendications 1 à 9, dans lequel on soumet ladite souche contenue dans le milieu de culture à l'alimentation en sucre et en substrat.

12. Procédé selon l'une des revendications 1 à 11, dans lequel la zone de réaction contient initialement une concentration en substrat de 0,5 à 40 g.l⁻¹ de volume réactionnel initial et on procède à l'alimentation en continue de ladite souche en substrat lorsque la concentration initiale en substrat est de 0,1 à 15 g.l⁻¹.

13. Procédé selon l'une des revendications 1 à 12, dans lequel la quantité de cellules mises en jeu par rapport au volume réactionnel est de 1 g à 100 g de poids sec par litre.

## Claims

1. Process for the fed batch production of a composition of sophorosides, in which culturing takes place of at least one Candida bombicola or Candida apicola strain in a culture medium including a nitrogen source under appropriate conditions for producing said strain and the cultured strain is exposed in a reaction zone to a sugar supply and a supply of at least one appropriate substrate under adequate aeration, temperature and pH conditions, characterized in that the following sequence is performed at least once:
a. the strain is continuously supplied with said substrate at a supply flow rate in the reaction zone between 0.01 and 4 grammes per hour and per litre of initial reaction volume and for a supply time such that the residual concentration of said substrate in the reaction zone is kept at a value at the most equal to 18 grammes per litre of initial reaction volume for said supply time and
b. the composition of sophorosides produced is recovered.

2. Process according to claim 1, wherein the sophoroside composition recovery stage comprises the stopping of the substrate supply or the aeration of the reaction zone, the recovery, after settling, of the lower sophoroside phase containing water and at least one washing with water of the sophorosides at a temperature between 10 and 90°C for 10 to 60 minutes.

3. Process according to either of the claims 1 and 2, wherein the substrate supply is stopped when the oxygen concentration in the reaction zone is substantially equal to 0.

4. Process according to either of the claims 1 and 2, wherein the substrate supply is stopped when the total injected substrate quantity reaches at the most 280 g·l⁻¹ of initial reaction volume.

5. Process according to any one of the claims 1 to 4, wherein sophoroside production takes place at a temperature of 18 to 35°C, at a pH of 2.5 to 8, in the presence of a sugar excess and in which the reaction zone is aerated at a rate of 0.2 to 2 v.v.m. under a pressure of 1 to 5 bar.

6. Process according to any one of the claims 1 to 5, wherein the substrate comprises at least one animal oil, at least one vegetable oil and/or at least one ester of said oil, said oils and said ester incorporating an aliphatic, straight chain with 10 to 24 carbon atoms.

7. Process according to any one of the claims 1 to 6, wherein the strain is Candida bombicola CBS 6009.

8. Process according to any one of the claims 1 to 7, wherein the culture medium comprises sugar.

9. Process according to any one of the claims 1 to 8, wherein the substrate flow rate into the reaction zone is between 1.0 and 3.0 g·l⁻¹·h⁻¹ of initial reaction volume.

10. Process according to any one of the claims 1 to 9, wherein the strain is obtained from an ex-situ prepared culture.

11. Process accoridng to any one of the claims 1 to 9, wherein the strain contained in the culture medium is exposed to the substrate and sugar supply.

12. Process according to any one of the claims 1 to 11, wherein the reaction zone initially contains a substrate concentration of 0.5 to 40 g·l⁻¹ of initial reaction volume and the substrate is continuously supplied to said strain when the initial substrate concentration is 0.1 to 15 g·l⁻¹ .

13. Process according to any one of the claims 1 to 12, wherein the cell quantity used, based on the reaction volume, is 1 g to 100 g of dry weight per litre.

## Patentansprüche

1. Verfahren zur Produktion im fed-batch eines Gemisches aus Sophorosiden, bei dem man mindestens einen Stamm *Candida bombicola* oder *Candida apicola* in einem eine Stickstofffquelle enthaltenden Kulturmedium unter geeigneten Bedingungen kultiviert, so daß man den besagten Stamm erzeugt und den besagten kultivierten Stamm in einer Reaktionszone mit einer Zufuhr von Zucker und einer Zufuhr von mindestens einem geeigneten Substrat unter angemessenen Bedingungen der Belüftung, der Temperatur und des pH-Werts unterwirft, dadurch gekennzeichnet, daß man mindestens einmal die folgende Sequenz durchführt:
a) die Ernährung des Stammes mit dem besagten Substrat wird in der Reaktionszone mit einer Ernährungszufuhr zwischen 0,01 und 4 Gramm pro Stunde und pro Liter des Anfangsreaktionsvolumens und während einer Ernährungsdauer, die so bemessen ist, daß die verbleibende Konzentration an dem besagten Substrat in der Reaktionszone auf einem Wert von mehr als 18 Gramm pro Liter des Ausgangsreaktionsvolumens während der besagten Ernährungsdauer gehalten wird, durchgeführt; und
b) das Gemisch der erzeugten Sophoroside gewonnen wird.

2. Verfahren nach Anspruch 1, bei dem der Schritt zur Gewinnung des Gemisches von Sophorosiden das Anhalten der Versorgung mit Substrat oder der Belüftung der Reaktionszone, die Gewinnung der unteren Wasser enthaltenden Sophorosidenphase nach dem Absetzenlassen und mindestens eine Wäsche der Sophorosiden mit Wasser bei einer Temperatur zwischen einschließlich 10 und 90 °C 10 bis 60 Minuten lang umfaßt.

3. Verfahren nach einem der Ansprüche 1 bis 2, bei dem die Versorgung mit Substrat angehalten wird, wenn die Sauerstoffkonzentration in der Reaktionszone fast gleich Null ist.

4. Verfahren nach einem der Ansprüche 1 bis 2, bei dem die Versorgung mit Substrat angehalten wird, wenn die Gesamtmenge des zugesetzten Substrates mehr als 280 g/l des Ausgangsreaktionsvolumens erreicht.

5. Verfahren nach einem der Ansprüche 1 bis 4, bei dem man die Herstellung der Sophorosiden bei einer Temperatur von 18 bis 35 °C, bei einem pH-Wert von 2,5 bis 8 und in Gegenwart eines Überschusses an Zucker durchführt und bei der man die Reaktionszone mit einem Durchsatz von Luft mit einer Raumgeschwindigkeit von 0,2 bis 2 Volumen pro Volumen und Minute unter einem Druck von 1 bis 5 bar versorgt.

6. Verfahren nach einem der Ansprüche 1 bis 5, bei dem das Substrat mindestens ein tierisches Öl, mindestens ein pflanzliches Öl und/oder mindestens einen Ester des besagten Öls enthält und der besagte Ester eine lineare aliphatische Kette mit 10 bis 24 Kohlenstoffatomen umfaßt.

7. Verfahren nach einem der Ansprüche 1 bis 6, bei dem der Stamm *Candida bombicola* CBS 6009 ist.

8. Verfahren nach einem der Ansprüche 1 bis 7, bei dem das Kulturmedium Zucker enthält.

9. Verfahren nach einem der Ansprüche 1 bis 8, bei dem der Substratdurchsatz in der Reaktionszone zwischen 1,0 und 3,0 g/l·h des Ausgangsreaktionsvolumens beträgt.

10. Verfahren nach einem der Ansprüche 1 bis 9, bei dem der Stamm aus einer ex-situ durchgeführten Kultur stammt.

11. Verfahren nach einem der Ansprüche 1 bis 9, bei dem man den besagten im Kulturmedium enthaltenen Stamm mit Zucker und Substrat versorgt.

12. Verfahren nach einem der Ansprüche 1 bis 11, bei dem die Reaktionszone ursprünglich eine Substratkonzentration von 0,5 bis 40 g/l des Ausgangsreaktionsvolumens enthält und man mit der kontinuierlichen Versorgung des besagten Stammes mit Substrat fortfährt, wenn die Ausgangskonzentration des Substrats zwischen 0,1 und 15 g/l liegt.

13. Verfahren nach einem der Ansprüche 1 bis 12, bei dem die Menge der eingesetzten Zellen bezogen auf das Reaktionsvolumen zwischen 1 bis 100 g Trockengewicht pro Liter liegt.
